# EUROPEAN PATENT APPLICATION

(11) **EP 4 134 445 A1**
(43) Date of publication of application: **15.02.2023**
(21) Application number: 21194880.7
(22) Date of filing: 03.09.2021
(51) Int. Cl.: C12Q 1/6876

(54) **METHOD FOR GENETIC SELECTION BASED ON MEAT QUALITY AND GROWTH**

(30) Priority: 13.08.2021 PT 2021117399
(71) Applicant: Cebal - Centro De Biotecnologia Agrícola E Agro-Alimentar Do Alentejo, 7800-309 Beja (PT); Universidade De Évora, 7004-516 Évora (PT); INIAV - Instituto Nacional de Investigação Agrária E Veterinária, 2780-157 Oeiras (PT)
(72) Inventor: COSTA DO AMARAL RAMOS, ANTÓNIO MARCOS, 7800-309 BEJA (PT); USIÉ CHIMENOS, ANA ISABEL, 7800-309 BEJA (PT); BRANCO GASPAR, DANIEL FILIPE, 7800-309 BEJA (PT); PINHEIRO RIBEIRO DE MEIRELES, BRÍGIDA DA NATIVIDADE, 2685-019 SACAVÉM (PT); CARVALHO MAGALHÃES, HUGO, 4700-284 BRAGA (PT); MOTA RUIVO MARTINS, JOSÉ MANUEL, 7005-401 ÉVORA (PT); SANTOS BARBOSA, PEDRO, 1000-127 LISBOA (PT); FIALHO LEÃO, CÉLIA CRISTINA, 2780-157 OEIRAS (PT); CARRACHA CHARNECA, RUI MIGUEL, 7005-401 ÉVORA (PT); SOUSA JERÓNIMO, ELIANA ALEXANDRA, 7800-309 BEJA (PT); SARGENTO CACHUCHO, LILIANA MARGARIDA, 7800-309 BEJA (PT); PIMENTEL CAROLINO, RENATO NUNO, 2005-048 VALE DE SANTARÉM (PT)
(74) Representative: Neves, Ana

(57) **Abstract**

The present patent application discloses a method for the genetic selection of animals from the Alentejano pig breed with a genetic profile associated with better meat quality and higher growth. Said profile is determined based on a group of Single Nucleotide Polymorphisms (SNPs) herein associated with better meat quality and higher growth in the Alentejano pig breed.

## Description

### Technical field

The present patent application relates to a method for the genetic selection of animals from the Alentejano pig breed with better meat quality and growth rates.

### Background art

The quality of pork meat is quite variable between pig breeds. Nowadays there are several pig breeds widely used in intensive pork production systems, where the quality of the meat observed is usually lower than that of native breeds, operated in extensive systems, and with distinct genetic characteristics. An example of this type of breeds is the Alentejano Pig, a Portuguese native breed, recognized for the high quality of the meat and products that are obtained from animals of this breed. Whether for fresh consumption or for the production of hams, paletas and sausages, it is essential to maximize the quality of the meat produced by these animals.

Carcass weight can be considered as the final stage of the growth potential presented by the animal during its productive life and is also one of the phenotypes of special relevance in the context of Alentejo Pig production.

To date, the studies focused on the identification of genetic markers associated with pork meat quality have focused mainly on the breeds most used in intensive production, there is no information of this kind for the Alentejo Pig breed. The same happens with growth, since to date there is no information available for genetic markers associated with this phenotype. On the other hand, genome sequencing of animals of the Porco Alentejano breed offers tremendous potential for determining the variation present in each animal analyzed. When this process is applied to groups of animals with contrasting phenotypic characteristics it is possible to identify the positions in the genome that are most different among each group of animals. In this way it is then possible to identify the most relevant SNPs that can later be used to select animals carrying the variants of greatest interest.

### Summary

The present patent application relates to a method for the genetic selection of animals from the Alentejano pig breed with better meat quality and higher growth rates

The method for selection of animals from the Alentejano pig breed based on genotypes associated with better meat quality and higher growth rates comprises the following steps:
Preparation of an Alentejano pig biological sample and extraction of DNA from said sample;
Genotyping the DNA sample for at least one of the following Single Nucleotide Polymorphisms (SNPs),

| Chromosome | Position | G1_FA0 | G1_FA1 | G2_FA0 | G2_FA1 | Difference |
|---|---|---|---|---|---|---|
| 1 | 2,319,234 | 1 | 0 | 0.4 | 0.6 | 0.6 |
| 1 | 2,346,404 | 1 | 0 | 0.458 | 0.542 | 0.542 |
| 1 | 77,043,987 | 0.75 | 0.25 | 0.167 | 0.833 | 0.583 |
| 1 | 222,837,705 | 0 | 1 | 0.577 | 0.423 | 0.577 |
| 1 | 222,849,410 | 0.042 | 0.958 | 0.591 | 0.409 | 0.549 |
| 1 | 222,854,205 | 0.038 | 0.962 | 0.583 | 0.417 | 0.545 |
| 1 | 222,921,800 | 0.038 | 0.962 | 0.583 | 0.417 | 0.545 |
| 1 | 222,940,782 | 0.042 | 0.958 | 0.583 | 0.417 | 0.541 |
| 1 | 239,892,759 | 0.909 | 0.091 | 0.375 | 0.625 | 0.534 |
| 2 | 86,965 | 0.833 | 0.167 | 0.278 | 0.722 | 0.555 |

Determining the pig's meat quality and growth based on the presence of at least one of said SNPs significantly associated with better meat quality and higher growth rates.

In one embodiment, the sample analyzed is preferably a blood sample.

In another embodiment, the above-mentioned Single Nucleotide Polymorphyms (SNPs) are used as biomarkers of better meat quality and higher growth rates in Alentejano pig breed animals.

### Detailed Description

The present patent application discloses a method for the genetic selection of animals from the Alentejano pig breed with profile associated with better meat quality and higher growth rates. Said profile is determined based on a group of Single Nucleotide Polymorphisms (SNPs) disclosed herein which are associated with better meat quality and higher growth rates in the Alentejano pig breed.

The technology disclosed herein is based on the sequencing of genomes of animals of the Alentejano Pig breed, to identify the global set of variations, namely SNPs, present in the genome of each animal. This sequencing is done on groups of animals with contrasting phenotype for meat quality and carcass weight. The next step involves determining the allele frequencies observed in each of the groups, and identifying the SNPs with the largest differences observed between the groups for the frequency of the alternative allele. Once the SNPs with the greatest difference in allele frequency were identified, it became possible to genotype these SNPs in other animals, selecting the animals carrying the most frequent alleles in the group of animals, with sequenced genome, that showed the best performances for meat quality and growth.

Ten SNPs are identified herein and associated with an improvement of meat quality and growth in Alentejano pig breed animals. The complete list of these SNPs is shown in Table 1.

The 10 SNPs identified are described in Table 1.

| Chromosome | Position | G1_FA0 | G1_FA1 | G2_FA0 | G2_FA1 | Difference | Gene |
|---|---|---|---|---|---|---|---|
| 1 | 2,319,234 | 1 | 0 | 0.4 | 0.6 | 0.6 | ENSSSCG00000004022 |
| 1 | 2,346,404 | 1 | 0 | 0.458 | 0.542 | 0.542 | ENSSSCG00000004022 |
| 1 | 77,043,987 | 0.75 | 0.25 | 0.167 | 0.833 | 0.583 | ENSSSCG00000004394 |
| 1 | 222,837,705 | 0 | 1 | 0.577 | 0.423 | 0.577 | ENSSSCG00000005250 |
| 1 | 222,849,410 | 0.042 | 0.958 | 0.591 | 0.409 | 0.549 | ENSSSCG00000005250 |
| 1 | 222,854,205 | 0.038 | 0.962 | 0.583 | 0.417 | 0.545 | ENSSSCG00000005250 |
| 1 | 222,921,800 | 0.038 | 0.962 | 0.583 | 0.417 | 0.545 | ENSSSCG00000005250 |
| 1 | 222,940,782 | 0.042 | 0.958 | 0.583 | 0.417 | 0.541 | ENSSSCG00000005250 |
| 1 | 239,892,759 | 0.909 | 0.091 | 0.375 | 0.625 | 0.534 | ENSSSCG00000005373 |
| 2 | 86,965 | 0.833 | 0.167 | 0.278 | 0.722 | 0.555 | ENSSSCG00000014565 |

The method of the present patent application involves several steps. Initially, it is necessary to collect a sample of biological material, such as a blood sample, for DNA extraction. Next, this DNA sample is genotyped for the SNPs of interest, and the genotypes obtained for each SNP are evaluated. The decision to select the animal is based on the presence of the genotype (s) significantly associated with better meat quality and higher growth rates.

The method for selection of animals from the Alentejano pig breed based on genotypes with better meat quality and highest growth rates comprises the following steps:
Preparation of an Alentejano pig biological sample and extraction of DNA from said sample;
Genotyping the DNA sample for at least one of the following Single Nucleotide Polymorphisms (SNPs),

| Chromosome | Position | G1_FA0 | G1_FA1 | G2_FA0 | G2_FA1 | Difference |
|---|---|---|---|---|---|---|
| 1 | 2,319,234 | 1 | 0 | 0.4 | 0.6 | 0.6 |
| 1 | 2,346,404 | 1 | 0 | 0.458 | 0.542 | 0.542 |
| 1 | 77,043,987 | 0.75 | 0.25 | 0.167 | 0.833 | 0.583 |
| 1 | 222,837,705 | 0 | 1 | 0.577 | 0.423 | 0.577 |
| 1 | 222,849,410 | 0.042 | 0.958 | 0.591 | 0.409 | 0.549 |
| 1 | 222,854,205 | 0.038 | 0.962 | 0.583 | 0.417 | 0.545 |
| 1 | 222,921,800 | 0.038 | 0.962 | 0.583 | 0.417 | 0.545 |
| 1 | 222,940,782 | 0.042 | 0.958 | 0.583 | 0.417 | 0.541 |
| 1 | 239,892,759 | 0.909 | 0.091 | 0.375 | 0.625 | 0.534 |
| 2 | 86,965 | 0.833 | 0.167 | 0.278 | 0.722 | 0.555 |

Determining the pig's meat quality and growth based on the presence of at least one of said SNPs significantly associated with better meat quality and higher growth rates.

In one embodiment, the sample analyzed is preferably a blood sample.

## Claims

1. Method for selection of animals from the Alentejano pig breed based on genotypes associated with better meat quality and higher growth comprising the following steps:
Preparation of an Alentejano pig biological sample and extraction of DNA from said sample;
Genotyping the DNA sample for at least one of the following Single Nucleotide Polymorphisms (SNPs),
| Chromosome | Position | G1_FA0 | G1_FA1 | G2_FA0 | G2_FA1 | Difference |
|---|---|---|---|---|---|---|
| 1 | 2,319,234 | 1 | 0 | 0.4 | 0.6 | 0.6 |
| 1 | 2,346,404 | 1 | 0 | 0.458 | 0.542 | 0.542 |
| 1 | 77,043,987 | 0.75 | 0.25 | 0.167 | 0.833 | 0.583 |
| 1 | 222,837,705 | 0 | 1 | 0.577 | 0.423 | 0.577 |
| 1 | 222,849,410 | 0.042 | 0.958 | 0.591 | 0.409 | 0.549 |
| 1 | 222,854,205 | 0.038 | 0.962 | 0.583 | 0.417 | 0.545 |
| 1 | 222,921,800 | 0.038 | 0.962 | 0.583 | 0.417 | 0.545 |
| 1 | 222,940,782 | 0.042 | 0.958 | 0.583 | 0.417 | 0.541 |
| 1 | 239,892,759 | 0.909 | 0.091 | 0.375 | 0.625 | 0.534 |
| 2 | 86,965 | 0.833 | 0.167 | 0.278 | 0.722 | 0.555 |
Determining the pig's meat quality and growth based on the presence of at least one of said SNPs significantly associated with better meat quality and higher growth rates.

2. Single Nucleotide Polymorphyms (SNPs) as defined in claim 1 for use as biomarkers of better meat quality and higher growth rates in Alentejano pig breed animals.
